# EUROPEAN PATENT APPLICATION

(11) **EP 3 901 613 A1**
(43) Date of publication of application: **27.10.2021**
(21) Application number: 19900044.9
(22) Date of filing: 19.12.2019
(51) Int. Cl.: G01N 21/00

(54) **METHOD AND SYSTEM FOR FORMULATING A REQUIRED COMPOSITION FROM AT LEAST ONE INGREDIENT OF VARIABLE COMPOSITION**

(30) Priority: 19.12.2018 MX 2018016129
(71) Applicant: Sigma Alimentos, S. A. De C. V., Nuevo León 66254 (MX)
(72) Inventor: PÉREZ GALLARDO, Alfonso, C.P. 66600, Apodaca, Nuevo León (MX); LUNA MARROQUÍN, Néstor, C.P. 67300, Villa de Santiago, Nuevo León (MX); DE HAENE ROSIQUE, Gregorio José, C.P. 64987 Monterrey, Nuevo Leon (MX)
(74) Representative: Codoñer Molina, Vicente
(86) International application number: PCT/MX2019/000141
(87) International publication number: WO 2020/130778

(57) **Abstract**

A method and system for formulating a required composition in order to produce a determined product from the supply of at least one ingredient that has a time-varying composition; the method has the steps of: (a) pre-establishing a final profile of components of the required composition; (b) providing a first ingredient and a second ingredient, where at least one of them has a time-varying composition; (c) determining an initial profile of components of the first ingredient and the second ingredient; (d) estimating an amount of the first ingredient and the second ingredient to be supplied; (e) supplying, simultaneously or sequentially, the estimated amount of the first ingredient and the second ingredient, and simultaneously, in-line and/or in real-time, generating a real-time profile of components of the amount of the first ingredient and the second ingredient as they are being supplied; (f) determining in-line or in real-time whether the amount of the first ingredient and/or the amount of the second ingredient has changed its composition when comparing the profiles; (g) adjusting the estimate of the amount of the first ingredient and/or of the second ingredient being supplied, if any of them has changed its composition; and repeating steps (e) to (g) until the required composition is achieved.

## Description

### TECHNICAL FIELD OF THE INVENTION

The present invention relates to methods and systems for formulating a composition. More specifically, the present invention relates to a method and system for formulating a suitable composition to make a determined product from ingredients whose composition varies over time.

### BACKGROUND OF THE INVENTION

At present, it is common to prepare compositions from the mixture or reaction of two or more ingredients of stable composition, in such a way that as the mixture or reaction is prepared, this is quantitatively and qualitatively analyzed in real-time in order to determine its composition, so that in a controlled and dosed way, amounts of one or another ingredient are added until as long as the mixture or reaction does not reach a certain objective composition, all within a determined volume or mass.

To carry out the above, in general, calibration curves are established for each of the constituents that must make up the target composition, these calibration curves are correlated in real-time, using partial least squares regression techniques, analysis of partial components, or multiple linear regression, with the composition of constituents of said mixture or reaction determined in a timely manner and in real-time by quantitative and qualitative spectral analysis techniques of optical transmission, optical reflectance, dispersion and fluorescence, such as mid-infrared spectroscopy (MIR) and near infrared spectroscopy (NIR). Examples of some embodiments of this type are described in US patents US5258620, US6639044, US8072596 and US8158175; in US patent application publications US20100285186 and US20150306555; and in PCT international patent application publications WO0017611 and WO2015040626.

The techniques described in these patent documents have the disadvantage that they consider that the resulting compositions start from ingredients with a stable composition, so if a suitable composition is required to make a specific product from ingredients whose composition varies over time it cannot be to elaborate it with these techniques.

It is therefore necessary to offer a method and system for formulating in real-time a suitable composition to make a determined product from ingredients whose composition or constitution varies over time.

### SUMMARY OF THE INVENTION

In order to overcome the disadvantages of the state of the art described above, it is an object of the present invention to offer a method for formulating a composition required to produce a determined product, the method contemplates the steps of: (a) pre-establishing a final profile of components of the required composition to produce the determined product; (b) providing a first ingredient and a second ingredient, wherein at least one of these ingredients has a time-varying composition; (c) determining an initial profile of components of the first ingredient and an initial profile of components of the second ingredient; (d) estimating an amount of the first ingredient and an amount of the second ingredient to be supplied to form a composition close to the required composition, wherein these amounts are estimated by correlating the initial profile of components of the first ingredient and the initial profile of components of the second ingredient with the pre-established file profile of components; (e) supplying, simultaneously or sequentially, the estimated amount of the first ingredient and the estimated amount of the second ingredient, and simultaneously, in-line and/or in real-time, generating a real-time profile of components of the amount of the first ingredient as supplied and a real-time profile of components of the amount of the second ingredient as supplied; (f) determining in-line or in real-time whether at least one of the amounts of the first ingredient and the second ingredient has changed its composition, wherein said variation of composition is determined by simultaneously correlating the real-time profile of components of the amount of the first ingredient being supplied and the real-time profile of components of the amount of the second ingredient being supplied with the pre-established final profile of components; (g) adjusting the estimate of the amount of the first ingredient and/or the estimate of the second ingredient being supplied, under the determination that at least one of said amount of the first ingredient and amount of the second ingredient has varied its composition; and (h) repeating steps (e) to (g) until the close composition reaches the required composition to produce the determined product.

It is also an object of the present invention to offer a system for formulating a required composition in order to produce a determined product according to the method of claim 1, the system is formed by a first container to contain a first ingredient, the first container includes an outlet; a second container to contain a second ingredient, the second container includes an outlet; wherein at least one of said first ingredient and second ingredient has a time-varying composition; a first feeding line in communication with the outlet of the first container; a second feeding line in communication with the outlet of the second container; a first chemical composition sensor in the first feeding line; a second chemical composition sensor in the second feeding line; a storage tank in communication with the first feeding line and the second feeding line; a controller in communication with the first feeding line and the second feeding line; and a memory in communication with the controller for pre-establishing a final profile of components of a required composition to produce the determined product; wherein the first feeding line and the second feeding line are controlled by the controller to allow one or more amounts of the first ingredient and the second ingredient to be supplied to the storage tank; the first feeding line and the second feeding line are adapted to transmit mass or volume data of the amounts of the first ingredient and the second ingredient being supplied; the first chemical composition sensor and the second chemical composition sensor are adapted to transmit online and/or real-time data to the controller; and the controller is adapted to receive data from the first feeding line and the second feeding line, and from the first chemical composition sensor and the second chemical composition to estimate the amount of the first ingredient and the amount of the second ingredient to be supplied by the first feeding line and the second feeding line to the storage tank and to determine a component profile of the amounts of the first ingredient and the second ingredient and to correlate these profiles with the final profile of components pre-established in the memory.

### BRIEF DESCRIPTION OF THE DRAWINGS

The characteristic details of the present invention will be apparent from the following detailed description considered in connection with the accompanying drawings, for the purpose of defining the invention but without limiting its scope. It should be understood, however, that the drawings are made solely as an illustration and not as a limiting definition of the invention, in which:
Figure 1 illustrates a system for formulating in real-time a fluid composition required to produce a determined product according to the present invention.
Figure 2 illustrates a flow chart of a method for formulating in real-time a composition required to produce a determined product according to the present invention.
Figure 3A illustrates a diagram of component concentration changes over time of a dairy ingredient as it empties from the tank (container) containing it, according to the present invention.
Figure 3B illustrates a diagram of evolution over time of a concentration of components of a dairy food product composition during a mixing process according to the present invention.
Figure 4A illustrates a diagram of feeding ingredients to a storage tank for preparing a whipped yogurt composition according to the present invention.
Figure 4B illustrates a diagram of changes of composition of the whipped yogurt being prepared according to Figure 4A in accordance with the present invention.
Figure 5A illustrates a diagram of feeding ingredients to a storage tank for preparing a yogurt for drinking composition according to the present invention.
Figure 5B illustrates a diagram of changes of composition of the yogurt for drinking being prepared according to Figure 5A in accordance with the present invention.
Figure 6A illustrates a diagram of feeding ingredients to a storage tank for preparing an indulgent yogurt composition according to the present invention.
Figure 6B illustrates a diagram of changes of composition of the indulgent yogurt being prepared according to Figure 6A in accordance with the present invention.
Figure 7A illustrates a diagram of target values and real values measured according to the method of the present invention for various batches of preparation of whipped yogurt composition.
Figure 7B illustrates a diagram of target values and real values measured according to the method of the present invention for various batches of preparation of yogurt for drinking composition.
Figure 7C illustrates a diagram of target values and real values measured according to the method of the present invention for various batches of preparation of indulgent yogurt composition.

### DETAILED DESCRIPTION OF THE INVENTION

The characteristic details of this invention are described in the following paragraphs, which are intended to define the invention, but without limiting its scope.

Under the context of the present description, the term "time-varying composition" means a composition that over time undergoes some phenomenon that alters its composition, chemical concentration and/or state of aggregation during industrial stages of storage, processing and/or transport; some examples of these phenomena are: synthesis, decomposition, addition, aggregation, substitution, stratification, decantation, precipitation, sedimentation, chemical reactions, oxidation, combustion, gas evolution, heat absorption, heat evolution, acidification, alkalization, evaporation, coagulation, flocculation, curdling, distillation, condensation, protein interactions, caramelization, enolization, isomeration, dehydration, thermal degradation, enzymatic modifications, gelatinization, retrogradation, viscosity changes, crystallization, covalent bonding, derivatization, hydrolysis, putrefaction, proteolysis, resynthesis, transacylation, deamidation, desulfurization, lipolysis, retrogradation.

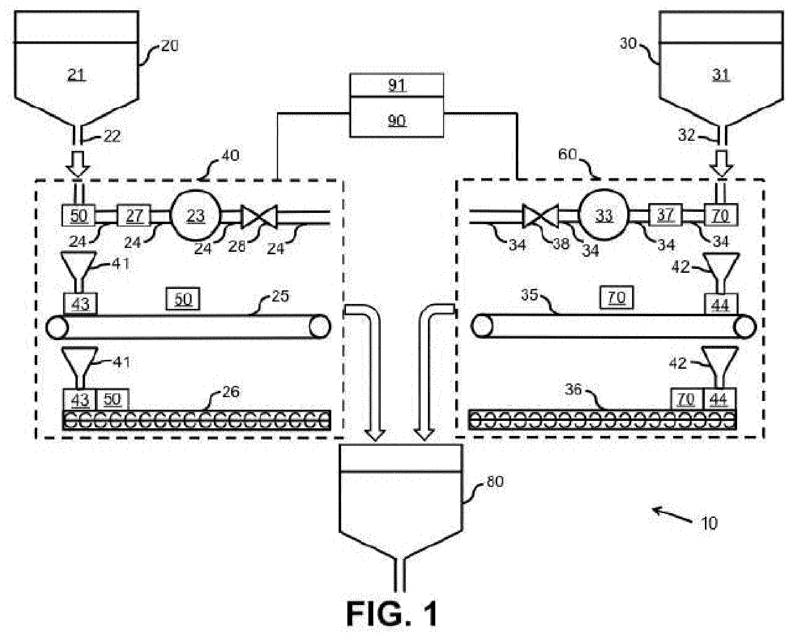

Figure 1 illustrates a system **10** for formulating in real-time a composition required to produce a determined product according to the present invention. The system **10** includes at least a first container **20,** a second container **30,** a first feeding line **40,** a first chemical composition sensor **50,** a second feeding line **60,** a second chemical composition sensor **70,** a storage tank **80** and a controller **90.**

The first container **20** and the second container **30** store a first ingredient **21** and a second ingredient **31,** respectively. The first ingredient and/or the second ingredient has a time-varying composition. The first ingredient **21** and the second ingredient **31** can be liquids, solids, powders and combinations thereof.

The first container **20** includes an outlet **22** in communication with the first feeding line **40,** while the second container **30** includes an outlet **32** in communication with the second feeding line **60.** The first feeding line **40** and the second feeding line **60** in turn are in communication with the storage tank **80.** In an alternative embodiment, the first feeding line **40** and the second feeding line **60** may be in communication with a mixing manifold (not shown) and this in turn in communication with storage tank **80.**

The first ingredient **21** and the second ingredient **31,** contained in the first container **20** and second container **30,** respectively, are supplied to the storage tank **80** through the first feeding line **40** and the second feeding line **60,** respectively.

In a first embodiment, when the first ingredient **21** and the second ingredient **31** are liquids, the first feeding line **40** and the second feeding line **60** may be made up of pumps **23** and **33,** respectively, arranged in or between conduits **24** and **34,** respectively. In a second and third embodiment, when the first ingredient **21** and the second ingredient 31 are solids or powders, the first feeding line **40** and/or the second feeding line **60** may be made up of conveyor belts **25** and **35,** respectively, or auger feeders **26** and **36,** respectively. In view of this, it is apparent to a person skilled in the art to carry out any combination of these embodiments of configurations of the first feeding line **40** and the second feeding line **60,** according to the type of first ingredient **21** and second ingredient **31** used in the present invention.

When using a system of pumps **23** and **33** as a configuration of the first feeding line **40** and/or the second feeding line **60,** in order to ensure adequate amounts of the first ingredient **21** and the second ingredient **31** in liquid state to be pumped to storage tank **80** or mixing manifold (not shown) use is made of meters **27** and **37** and valves **28** and **38** which may be upstream or downstream of pumps **23** and **33,** respectively. The meters **27** and **37** preferably measure mass or volume so that a precise amount of first ingredient **21** and/or second ingredient **31** flows into storage tank 80 or mixing manifold (not shown). The meters **27** and **37** can measure, respectively, the volume of the first ingredient **21** and the second ingredient **31** that are pumped, but can also measure other properties, such as mass or flow rate. The valves **28** and **38** can be of any type and can include a regulator that controls the rate and/or pressure of the flow of first ingredient **21** and/or second ingredient **31.** The valves **28** and **38** control flow rates and/or flow. pressure of the first ingredient **21** and/or of the second ingredient **31** to ensure that the velocities and/or pressures of these at the moment of being fed to the storage tank **80** or to the mixing manifold (not shown) correspond to an adequate flow.

At the outlet **22** of the first container **20** and at the outlet **32** of the second container **30** or in or between the respective conduits **24** and **34** are located the first chemical composition sensor **50** and the second chemical composition sensor **70,** respectively, however, in an alternative embodiment, the first chemical composition sensor **50** and the second chemical composition sensor **70** may be before or after the pumps **23** and **33,** respectively. The first chemical composition sensor **50** and the second chemical composition sensor **70** make it possible to determine, in real-time, a component profile of the first ingredient **21** and the second ingredient **31.**

The pumps **23** and **33** and the valves **28** and **38** are activated and controlled by the controller **90** based on the amounts of the first ingredient **21** and second ingredient 31 that in real-time are determined, according to the method of the present invention, to be fed to storage tank **80** or mixing manifold (not shown). The meters **27** and **37** transmit to the controller **90** data corresponding to the mass or volume of the amounts of the first ingredient **21** and the second ingredient **31** being fed to the storage tank **80** or to the mixing manifold (not shown), while the first chemical composition sensor **50** and the second chemical composition sensor **70** transmit data to the controller 90 to determine the profile of components of the first ingredient **21** and the second ingredient **31,** respectively, and then these data are used by the controller **90** to control the pumps **23** and **33,** and valves **28** and **38** throughout system **10** to ensure that precise chemical compositions and concentrations of the amounts determined and fed of the first ingredient **21** and the second ingredient **31** are used to mix and form a composition required to produce a determined product. These data transmissions from meters **27** and **37** and from the first chemical composition sensor **50** and the second chemical composition sensor **70** to the controller **90** and the activation and control of the pumps **23** and **33,** and the valves **28** and 38 by of the controller **90** are carried out through the use of electrical and/or electronic or radio frequency signals, so that there is electrical, electronic, optical or electromagnetic communication between these components of the system **10.**

When using a conveyor system **25** and **35** or auger feeder **26** and **36** as the configuration of the first feeding line **40** and/ or the second feeding line 60, in order to ensure adequate amounts of the first ingredient **21** and the second ingredient **31** in solid or powder state to be transported to the storage tank **80** or to the mixing manifold (not shown), use is made of dosing hoppers **41** and **42** in communication with the outlet **22** of the first container **20** and at the outlet **32** of the second container **30,** respectively and of mass meters **43** and **44** located in outlets of the dosing hoppers **41** and **42.** The mass meters **43** and **44** can be scales or load sensors and measure, respectively, the amount of mass of the first ingredient **21** and second ingredient **31** being dosed. The first chemical composition sensor **50** and the second chemical composition sensor **70** are located on the conveyor belts **25** and **35** or at the entrance of the auger feeders **26** and **36.** The first chemical composition sensor **50** and the second chemical composition sensor **70** allow determining, in real-time, a profile of components of the first ingredient **21** and the second ingredient **31,** respectively.

The speed of advance of the conveyors belts **25** and **35** or of the auger feeders **26** and **36** and the opening and closing of the dosing hoppers **41** and **42** are controlled by the controller **90** based on the amounts of the first ingredient **21** and second ingredient **31** that are determined in real-time, according to the method of the present invention, to be transported to the storage tank **80** or the mixing manifold (not shown). The mass meters **43** and **44** transmit to the controller **90** data corresponding to the mass of the amounts of the first ingredient **21** and the second ingredient **31** being transported to the storage tank **80** or to the mixing manifold (not shown), while the first chemical composition sensor **50** and the second chemical composition sensor **70** transmit data to controller **90** to determine the profile of components of the first ingredient **21** and the second ingredient **31,** respectively, and then these data are used by the controller **90** to control the conveyor belts **25** and **35** or auger feeders **26** and **36** and dosing hoppers **41** and **42** throughout the system **10** to ensure precise chemical compositions and concentrations of the amounts determined and fed of the first ingredient **21** and the second ingredient **31** are used to mix and form a composition required to produce a determined product. These data transmissions from the mass meters **43** and **44** and the first chemical composition sensor **50** and the second chemical composition sensor **70** towards the controller **90** and the activation and control of the conveyor belts **25** and **35** or the auger feeders **26** and **36** and the dosing hoppers **41** and **42** by the controller **90** are made by using electrical and/or electronic signals, so there is electrical and/or electronic communication between these components of the system **10.**

The first chemical composition sensor **50** and the second chemical composition sensor **70** can be an electrochemical transducer, optical transducer, mass transducer, thermal transducer, acoustic transducer, and combinations thereof; among the optical transducers are those of optical transmission, optical reflectance, dispersion, fluorescence and their combinations, either near infrared spectroscopy, visible light spectroscopy, fluorescence spectroscopy, Raman spectroscopy, surface-enhanced Raman spectroscopy (SERS), spectroscopy of other wavelengths within the magnetic spectrum, magnetic resonance and combinations thereof. Some examples of chemical composition sensors available in the state of the art and applicable to the present invention are listed in Table 1.

**Table 1**

| Denomination or trade name | Analysis parameter | Method or functional principle | Manufacturing company |
|---|---|---|---|
| Measurement of humidity in the process | Humidity | Microwave humidity sensor | PCE Instruments / Aquar System Ltd |
| Source Technology | Humidity | Resonance Hot oven | Source technology |
| | Protein and fat | NIR | |
| Online measurement of particle concentrations | Humidity y solids | Ultrasound LS-SVM | Unidentified |
| Milktronics | Turbidity | Optical | Milktronics |
| | Viscosity | Online viscometer | |
| | Solids | Optical / osmosis | |
| PendoTECH | Protein | Continuous flow UV sensor | PendoTECH |
| BioPAT®Spectro | Multiparameter | Flexible set of detectors for UV / VIS and NIR spectra | Sartorius STEDIM |
| NIR QUEST | Multiparameter | Configurable NIR / Spectrometer with configurable combs | Ocean OPTICS |
| Milkostream | Single point multiparameter for low viscosity fluids only | FTIR | FOSS |
| Milk-Inspector | Multiparameter | NIR | Quality2Process B.V. J&M Analytik AG |
| Unidentified | Lactose | Enzyme sensor - Cellobiose dehydrogenase based sensor | Lund University |
| Opti-I Relative Turbidity Sensor | Solids and Turbidity | NIR | Metrom instruments |
| THz | Hz spectroscopy has potential for the | Terahertz spectroscopy | Unidentified |
| | quantitative analysis of milk fat, total solid, lactose, milk protein, casein, and somatic cells. | | |
| Conductivity | Salts, solids, cryoscopic point (indirect) | Conductivity | Unidentified |
| Bio-transducers (Nanosensors, biosensors) | Salts, proteins, carbohydrates, toxins, contaminants, pathogens or viruses | Impedance Amperometry Potentiometry Cond uctometry Optical Raman spectroscopy SERS spectroscopy | Unidentified |

The controller **90** can be any programmable device that is pre-programmed, programmable by an operator, programmed and reprogrammed by a closed-loop logic system or preferably all of these are available in a single controller **90.** The controller **90** has an integrated memory **91** and may have different user interfaces, such as keyboards, displays, touchscreens, switches, audible sound generators, and others.

The controller **90** is preferably a Central Processing Unit ("CPU") or another programmable device such as a Printed Circuit Board ("PCB") to control, according to the configuration adopted of the first feeding line **40** and/or second feeding line **60,** pumps **23** and **33,** valves **28** and **38,** meters **27** and **37,** conveyor belts **25** and **35,** auger feeders **26** and **36,** dosing hoppers **41** and **42,** as well as receiving the information coming from meters **27** and **37,** mass meters **43** and **44,** the first chemical composition sensor **50** and the second chemical composition sensor **70.** A composition required to produce a determined product is preferably programmed into the controller **90** in order to control the amounts, feed rates, conveying rates and pressures with which the first ingredient **21** and/or second ingredient **31** must be fed to the storage tank **80** or the mixing manifold ado (not shown).

The system **10,** in particular the first feeding line **40** and the second feeding line **60,** can include a set of instruments to acquire process variables such as probes, spectrometers, transducers, thermocouples, pressure meters, density meters, flow meters, pH meters, among others (not shown).

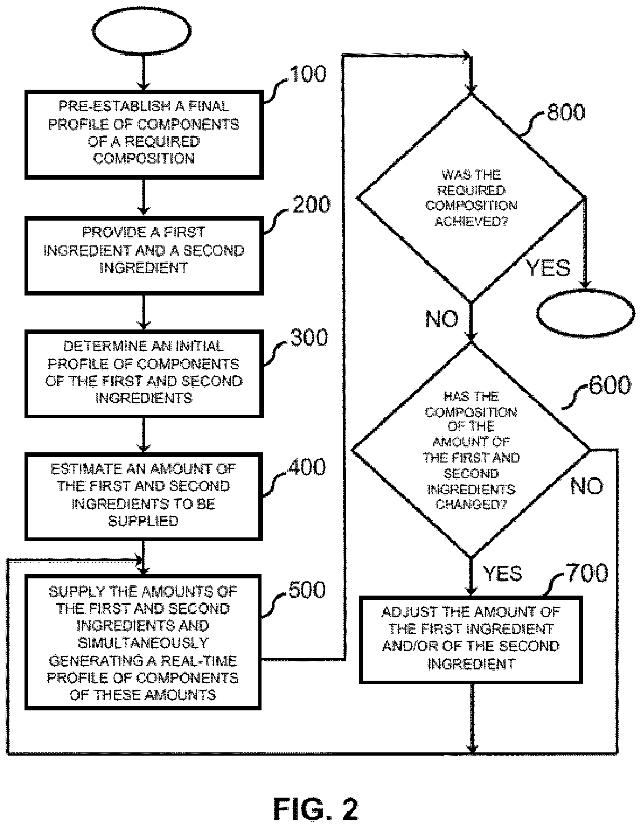

Now with reference to Figure 2, a method for formulating in real-time a composition required to produce a determined product according to the present invention is described below in relation to the system **10** of Figure 1.

The method can start in step **100,** where a final component profile of a composition required to produce a determined product is pre-established in memory **91** of controller **90,** this final component profile is generally determined and established by the using calibration curves for each of the constituents that must make up the composition required to produce a determined product, these calibration curves can be prepared as described in patent US5258620, the content of which is incorporated by reference.

At step **200,** first container **20** and second container **30** provide a first ingredient **21** and a second ingredient **31,** respectively, such that either or both of the first ingredient **21** and second ingredient **31** have a time-varying composition, due to any of the phenomena mentioned above.

Then in step **300** an initial profile of components of the first ingredient **21** and an initial profile of components of the second ingredient **31** are determined in the controller **90,** by means of a transduction that the first chemical composition sensor **50** performs to the first ingredient **21** and that the second chemical composition sensor **70** performs to the second ingredient **31,** the transduction information from the first chemical composition sensor **50** and the second chemical composition sensor **70** are transmitted to the controller **90** for processing in order to correlate it and thus determine the initial profile of components of the first ingredient **21** and the initial component profile of the second ingredient **31.**

Once the initial component profile of the first ingredient **21** and the initial component profile of the second ingredient **31** have been determined, the same controller **90,** in step **400,** estimates, by means of a mass balance, an amount of the first ingredient **21** and an amount of the second ingredient **31** to be supplied to form a composition close to the composition required to produce the determined product, these amounts are estimated by correlating the initial component profile of the first ingredient **21** and the initial component profile of the second ingredient **31** with the final profile of components pre-established in memory **91.**

Then, in step **500,** the controller **90** orders to the first feeding line **40** and the second feeding line **60** to supply, simultaneously or sequentially, the amount of the first ingredient **21** and the amount of the second ingredient **31** estimated, and simultaneously, online and/or in real-time, generate a real-time profile of components of the amount of the first ingredient **21** as it is supplied and a real-time profile of components of the amount of the second ingredient **31** as it is supplied, this through a online and/or real-time transduction that the first chemical composition sensor **50** performs on the amount of the first ingredient **21** as it is being supplied and that the second chemical composition sensor **70** performs on the amount of the second ingredient **31** as it is supplied, the transduction information from the first chemical composition sensor **50** and the second chemical composition sensor **70** is transmitted online and/or in real-time to the controller **90** for its processing in order to determine online or in real-time, in step 600, if one or both of said amount of the first ingredient **21** and amount of the second ingredient **31** has varied its composition by simultaneously correlating the real-time profile of components of the amount of the first ingredient **21** and the real-time profile of components of the amount of the second ingredient **31** with final profile of components pre-established in step **100.**

In case that the controller **90** determines that either or both of the amount of the first ingredient **21** and the amount of the second ingredient **31** has varied its composition, then the controller **90,** in step **700,** adjusts the estimate of the amount of the first ingredient **21** and/or the amount of the second ingredient **31** being supplied, ordering to the first feeding line **40** and/or the second feeding line **60** to supply the amount of the first ingredient **21** and/or the amount of the second ingredient **31** which have been suitable, and as long as the close composition does not reach the required composition to produce the determined product, step **800,** proceed with the repetition of steps **500, 600, 700** and **800.**

### EXAMPLES OF EMBODIMENT OF THE INVENTION

The invention will now be described with respect to the following examples, which are solely for the purpose of representing the manner of carrying out the implementation of the principles of the invention. The following examples are not intended to be an exhaustive representation of the invention, nor are they intended to limit the scope of the invention.

The examples that are illustrated below are applications of the principles described above of the present invention to prepare compositions of dairy food products, however it will be evident for a person skilled in the art to apply said principles of the invention in the preparation of any other composition in any technical field of application.

### EXAMPLE 1:

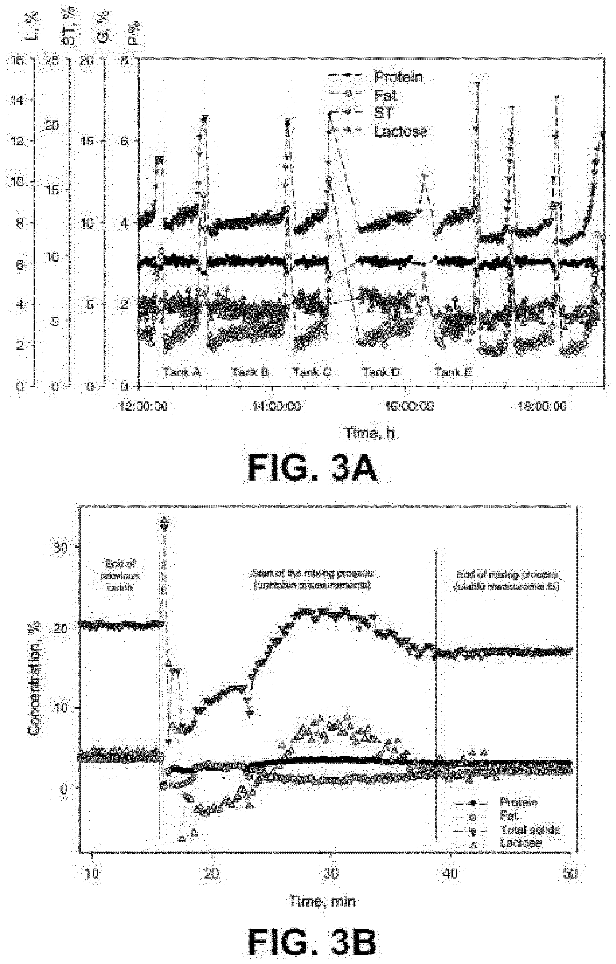

In relation to Figure 3A, it shows a diagram of a stratification phenomenon that a dairy ingredient undergoes as it is emptied from the tank (container) that contains in order to prepare a composition required for a dairy food product, thus observing the changes in the concentration of each of the components of the dairy ingredient over time, where "G" is fat, "L" is lactose, "P" is protein, and "ST" is total solids; while Figure 3B shows a diagram of evolution over time of the concentration of components of the composition required for a dairy food product being prepared by applying the method and system of the present invention.

### EXAMPLE 2:

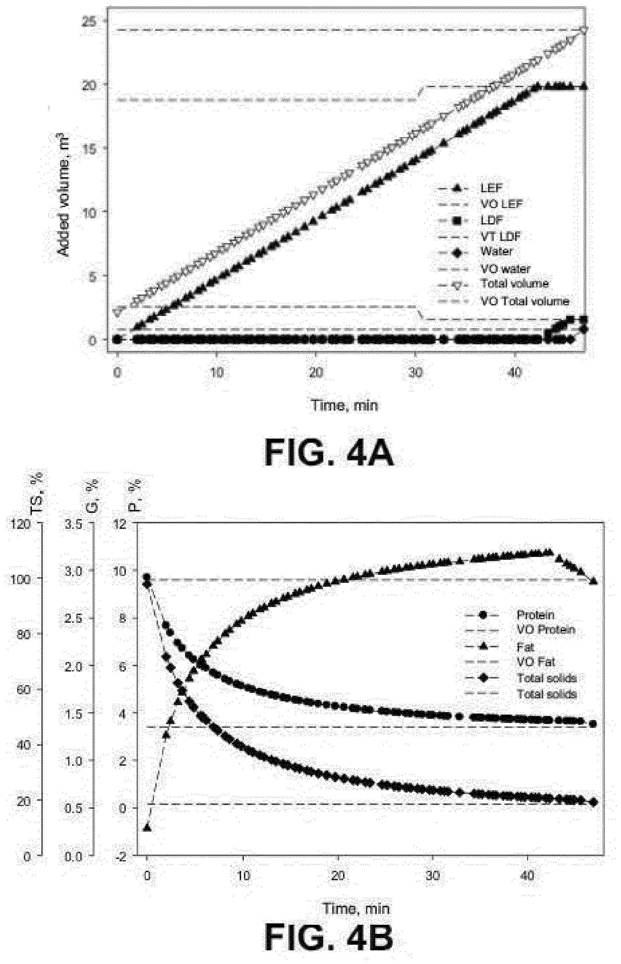

A composition of whipped yogurt was prepared, using as ingredients water, fluid whole milk (LEF) and fluid skim milk (LDF) that were fed according to the method and system of the invention to the storage tank as shown in the diagram of the Figure 4A, wherein it is also observed the target value of whole skimmed milk (VO LEF), the total volume of fluid skim milk (VT LDF), target value of water (VO water), total volume and the target value of the total volume (VO total volume); while the composition changes of whipped yogurt during the filling of the storage tank is shown in the diagram of Figure 4B, wherein the components shown are protein (P), fat (G) and total solids (TS) with respect to the target value of protein (VO protein), target value of fat (fat VO) and total solids, respectively. The desired target values (VO) and the real values (measured by methods of the present invention) are shown in Figures 4A and 4B, with very tight control of the required composition.

### EXAMPLE 3:

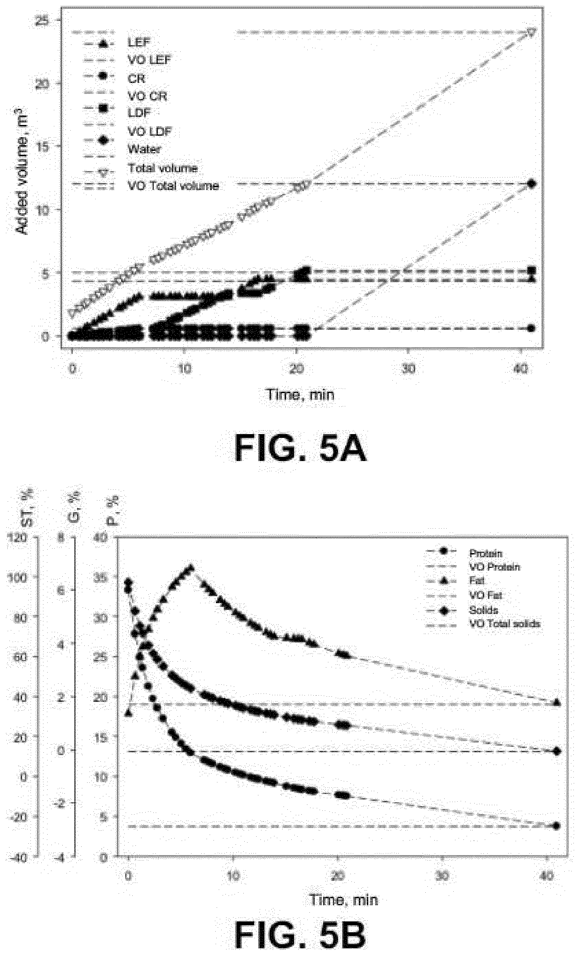

A composition of yogurt for drinking was prepared, using as ingredients water, fluid whole milk (LEF), fluid skim milk (LDF) and cream (CR) that were fed according to the method and system of the invention to the storage tank according to shown in the diagram of Figure 5A, wherein it is also observed the target value of whole skimmed milk (VO LEF), the total volume of fluid skim milk (VT LDF), target value of water (VO water), target value of cream (VO CR), total volume and the target value of total volume (VO total volume); while the composition changes of yogurt for drinking during the filling of the storage tank is shown in the diagram of Figure 5B, wherein the components shown are protein (P), fat (G) and total solids (TS) with respect to the target value of protein (VO protein), target value of fat (fat VO) and target value of total solids (total solid VO), respectively. The desired target values (VO) and the real values (measured by methods of the present invention) are shown in Figures 5A and 5B, with very close control of the required composition.

### EXAMPLE 4:

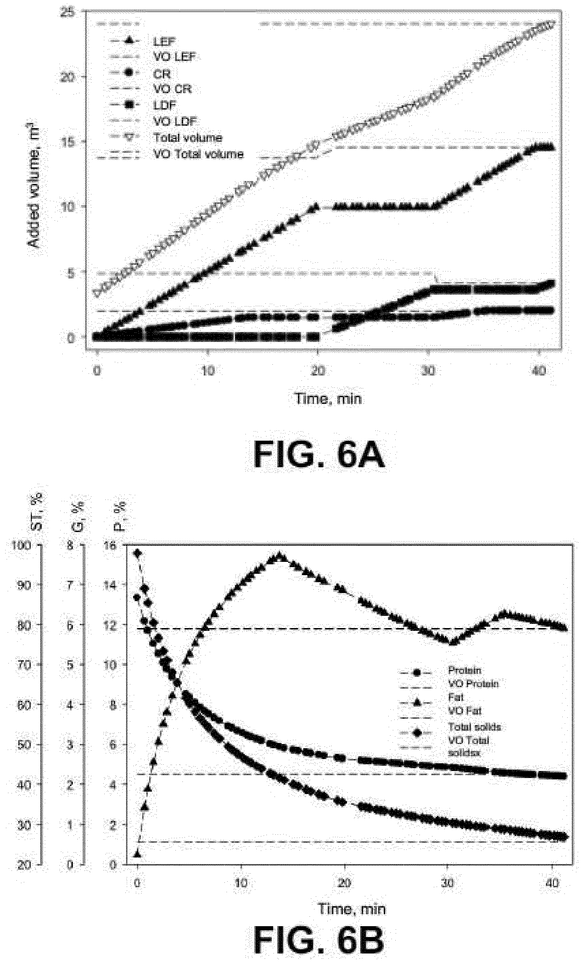

An indulgent yogurt composition was prepared, using as ingredients fluid whole milk (LEF), fluid skim milk (LDF) and cream (CR) that were fed according to the method and system of the invention to the storage tank as shown in the diagram of Figure 6A, wherein it is also observed the target value of whole skimmed milk (VO LEF), the total volume of fluid skim milk (VT LDF), target value of cream (VO CR), total volume and the target value of the total volume (VO total volume); while the composition changes of indulgent yogurt during the filling of the storage tank is shown in the diagram of Figure 6B, wherein the components shown are protein (P), fat (G) and total solids (TS) with respect to the target value of protein (VO protein), target value of fat (fat VO) and target value of total solids (total VO solids), respectively. The desired target values (VO) and the real values (measured by methods of the present invention) are shown in Figures 6A and 6B, with very close control of the required composition.

As seen in Examples 3, 4 and 5, after the mass balance calculation, the method and system of the present invention supplies the volume of liquid ingredients to the storage tank. During the filling of the storage tank to formulate the required composition, the method and system of the present invention controls the correct amount of each ingredient that must be supplied through the feeding lines, every time it is recalculated (adjusted) in real-time the amount of each ingredient being supplied when a change in the composition of any of these is detected.

### EXAMPLE 5:

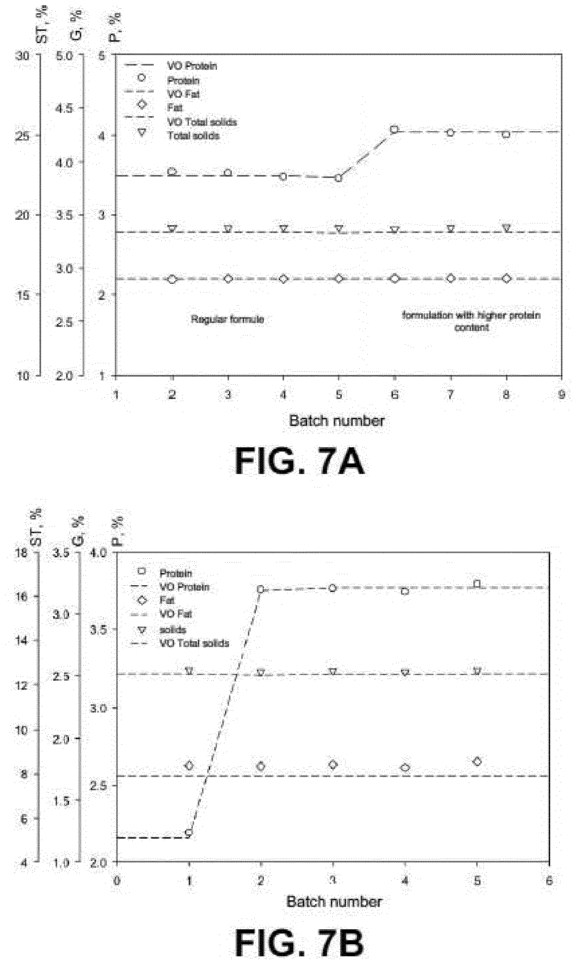

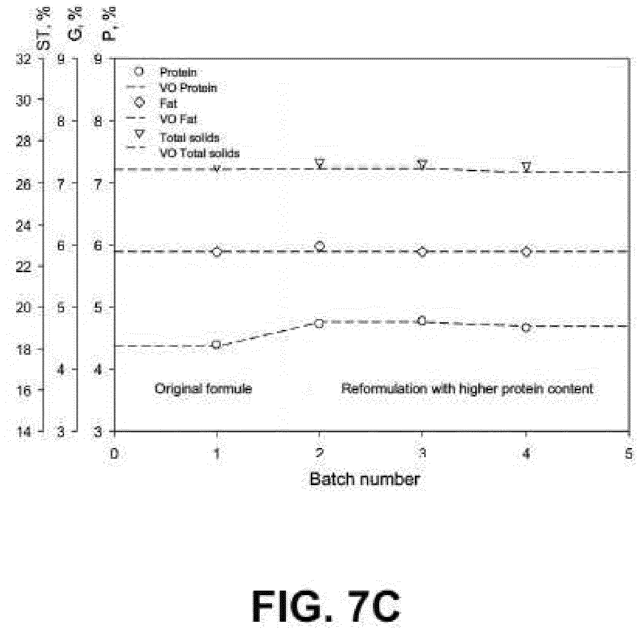

Figures 7A, 7B and 7C illustrate diagram of target values and real values measured according to the method of the present invention for various batches of composition preparation of whipped yogurt, yogurt for drinking and indulgent yogurt, respectively, wherein the components shown are protein the (P), fat (G) and total solids (TS) with respect to the target value of protein (VO protein), target value of fat (VO fat) and target value of total solids (VO total solids), respectively, for 5 batches of compositions, so it is observed that the method and system of the present invention allow a very close control of the composition.

Based on the embodiments described above, it is contemplated that modifications to the described embodiments, as well as alternative embodiments, will be apparent to a person skilled in the art under the present disclosure. It is therefore contemplated that the claims encompass such modifications and alternatives that are within the scope of the present invention or its equivalents.

## Claims

1. A method for formulating a required composition in order to produce a determined product, the method comprises of the steps of:
(a) pre-establishing a final profile of components of the required composition to produce the determined product;
(b) providing a first ingredient and a second ingredient, wherein at least one of these ingredients has a time-varying composition;
(c) determining an initial profile of components of the first ingredient and an initial profile of components of the second ingredient;
(d) estimating an amount of the first ingredient and an amount of the second ingredient to be supplied to form a composition close to the required composition, wherein these amounts are estimated by correlating the initial profile of components of the first ingredient and the initial profile of components of the second ingredient with the pre-established file profile of components;
(e) supplying, simultaneously or sequentially, the estimated amount of the first ingredient and the estimated amount of the second ingredient, and simultaneously, in-line and/or in real-time, generating a real-time profile of components of the amount of the first ingredient as supplied and a real-time profile of components of the amount of the second ingredient as supplied;
(f) determining in-line or in real-time whether at least one of the amounts of the first ingredient and the second ingredient has changed its composition, wherein said variation of composition is determined by simultaneously correlating the real-time profile of components of the amount of the first ingredient being supplied and the real-time profile of components of the amount of the second ingredient being supplied with the pre-established final profile of components;
(g) adjusting the estimate of the amount of the first ingredient and/or the estimate of the second ingredient being supplied, under the determination that at least one of said amount of the first ingredient and amount of the second ingredient has varied its composition; and
(h) repeating steps (e) to (g) until the close composition reaches the required composition to produce the determined product.

2. The method of claim 1, wherein the first ingredient and the second ingredient are selected from a group consisting of liquid ingredients, solid ingredients, powered ingredients and combinations thereof.

3. The method of claim 1, wherein the initial profile of components and the real-time profile of components are determined by transduction selected from the group consisting of electrochemical transduction, optical transduction, mass transduction, thermal transduction, acoustic transducer and combinations thereof.

4. The method of claim 3, wherein the optical transduction is selected from the group consisting of optical transmission, optical reflection, dispersion, fluorescence, and combinations thereof.

5. A system for formulating a required composition in order to produce a determined product according to the method of claim 1, the system comprises:
a first container to contain a first ingredient, the first container includes an outlet;
a second container to contain a second ingredient, the second container includes an outlet;
wherein at least one of said first ingredient and second ingredient has a time-varying composition;
a first feeding line in communication with the outlet of the first container;
a second feeding line in communication with the outlet of the second container;
a first chemical composition sensor in the first feeding line;
a second chemical composition sensor in the second feeding line;
a storage tank in communication with the first feeding line and the second feeding line;
a controller in communication with the first feeding line and the second feeding line; and
a memory in communication with the controller for pre-establishing a final profile of components of a required composition to produce the determined product;
wherein the first feeding line and the second feeding line are controlled by the controller to allow one or more amounts of the first ingredient and the second ingredient to be supplied to the storage tank; the first feeding line and the second feeding line are adapted to transmit mass or volume data of the amounts of the first ingredient and the second ingredient being supplied; the first chemical composition sensor and the second chemical composition sensor are adapted to transmit online and/or real-time data to the controller; and the controller is adapted to receive data from the first feeding line and the second feeding line, and from the first chemical composition sensor and the second chemical composition to estimate the amount of the first ingredient and the amount of the second ingredient to be supplied by the first feeding line and the second feeding line to the storage tank and to determine a component profile of the amounts of the first ingredient and the second ingredient and to correlate these profiles with the final profile of components pre-established in the memory.

6. The system of claim 5, wherein the first ingredient and the second ingredient are selected from a group consisting of liquid ingredients, solid ingredients, powered ingredients and combinations thereof.

7. The system of claim 5, wherein the first feeding line and the second feeding line are selected from a group consisting of pumps, conveyors, auger feeders and combinations thereof.

8. The system of claim 5, wherein the first chemical composition sensor and the second chemical composition sensor is a transducer selected from the group consisting of electrochemical transducer, optical transducer, mass transducer, thermal transducer, acoustic transducer and combinations thereof.

9. The system of claim 8, wherein the optical transducer is selected from the group consisting of optical transmission transducer, optical reflectance transducer, dispersion transducer, fluorescence transducer and combinations thereof.
